(19) Europäisches Patentamt
European Patent Office
Office européen des brevets

(11) Veröffentlichungsnummer: **0 254 797**
**B1**

(12) # EUROPÄISCHE PATENTSCHRIFT

(45) Veröffentlichungstag der Patentschrift: **04.07.90**

(21) Anmeldenummer: **87102578.9**

(22) Anmeldetag: **24.02.87**

(51) Int. Cl.[5]: **C 07 D 249/08,**
A 01 N 43/653,
C 07 D 303/22, C 07 C 49/255

(54) **2-Hydroxyethyl-azol-Derivate.**

(30) Priorität: **06.03.86 DE 3607286**

(43) Veröffentlichungstag der Anmeldung:
**03.02.88 Patentblatt 88/05**

(45) Bekanntmachung des Hinweises auf die Patenterteilung:
**04.07.90 Patentblatt 90/27**

(84) Benannte Vertragsstaaten:
**AT BE CH DE FR GB IT LI NL**

(56) Entgegenhaltungen:
**EP-A-0 040 345**
**EP-A-0 040 350**
**EP-A-0 052 424**
**EP-A-0 084 834**
**EP-A-0 150 036**

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Lantzsch, Reinhard, Dr.**
**Am Buschhaeuschen 51**
**D-5600 Wuppertal 11 (DE)**
Erfinder: **Brandes, Wilhelm, Dr.**
**Eichendorffstrasse 3**
**D-5653 Leichlingen (DE)**
Erfinder: **Hänssler, Gerd, Dr.**
**Am Arenzberg 58a**
**D-5090 Leverkusen 3 (DE)**
Erfinder: **Reinecke, Paul, Dr.**
**Steinstrasse 8**
**D-5090 Leverkusen 3 (DE)**
Erfinder: **Lürssen, Klaus, Dr.**
**August-Kierspel-Strasse 151**
**D-5060 Bergisch-Gladbach 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 254 797 B1

**Beschreibung**

Die vorliegende Erfindung betrifft neue 2-Hydroxyethylazol-Derivate, ein Verfahren zu deren Herstellung und deren Verwendung als Fungizide und Pflanzenwachstumsregulatoren. Die Erfindung betrifft außerdem Oxirane und phenoxy-substituierte Ketone, Verfahren zu deren Herstellung und deren Verwendung als Zwischenprodukte zur Synthese von Stoffen mit fungizider und pflanzenwuchsregulierender Wirksamkeit.

Es ist bereits bekannt, daß zahlreiche 2-Hydroxyethylazol-Derivate fungizide und pflanzenwuchsregulierende Eigenschaften besitzen (vgl. EP—OS 0 040 345). So kann zum Beispiel 1-Phenoxy-3,3-dimethyl-2-(1,2,4-triazol-1-yl-methyl)-butan-2-ol zur Bekämpfung von Pilzen und zur Regulierung des Pflanzenwachstums eingesetzt werden. Bei niedrigen Aufwandmengen läßt die Wirkung dieses Stoffes in manchen Fällen allerdings zu wünschen übrig.

Weiterhin ist bereits bekannt, daß sich Phenoxypropylketone herstellen lassen, indem man Phenoxypropyl-Grignard-Verbindungen mit Aldehyden umsetzt und die dabei entstehenden Carbinole dann oxidiert (vgl. EP—OS 0 150 404). Nachteilig für eine Durchführung dieses Verfahrens im technischen Maßstab ist jedoch, daß im Rahmen der Synthese metallorganische Verbindungen benötigt werden. Ungünstig ist ferner, daß die als Ausgangsmaterialien erforderlichen Aldehyde zum Teil nur schlecht zugänglich sind. Ferner muß als weiterer Verfahrensschritt eine Oxidation der Hydroxygruppe vorgenommen werden. Dieses ist insbesondere dann ungünstig, wenn die betreffende Ketogruppe in den Endprodukten, die aus den Phenoxyproplketonen hergestellt werden, wieder zu einer Carbinolgruppe reduziert werden muß (vgl. DE—OS 3 019 049 und DE—OS 3 209 431).

Es wurden nun neue 2-Hydroxyethyl-azol-Derivate der Formel

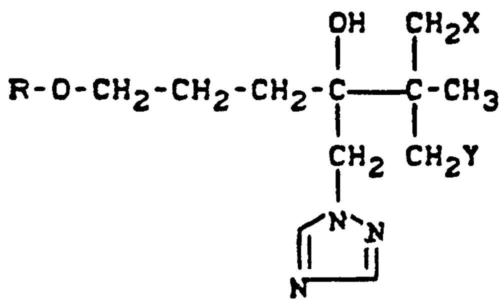

(I)

in welcher

R für gegebenenfalls einfach bis dreifach, gleichartig oder verschieden substituiertes Phenyl steht, wobei als Substituenten in Frage kommen; Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Cycloalkyl mit 3 bis 7 Kohlenstoffatomen, Phenyl, Phenoxy, und Alkoximinoalkyl mit 1 bis 4 Kohlenstoffatomen in der Alkoxygruppe und 1 bis Kohlenstoffatomen in der Alkylgruppe,

X für Wasserstoff, Fluor, Chlor oder Brom steht und

Y für Wasserstoff, Fluor, Chlor oder Brom steht,

sowie deren Säureadditions-Salze und Metallsalz-Komplexe gefunden.

Weiterhin wurde gefunden, daß man 2-Hydroxyethyl-azol-Derivate der Formel (I) sowie deren Säureadditions-Salze und Metallsalz-Komplexe erhält, wenn man Oxirane der Formel

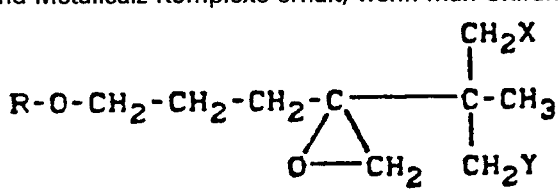

(II)

in welcher

R, X und Y die oben angegebene Bedeutung haben, mit 1,2,4-Triazol der Formel

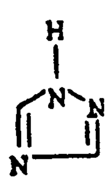

(III)

in Gegenwart eines Verdünnungsmittels und gegebenenfalls in Gegenwart einer Base umsetzt und

gegebenenfalls anschließend an die so erhaltenen Verbindungen der Formel (I) eine Säure oder ein Metallsalze addiert.

Außerdem wurde gefunden, daß die neuen 2-Hydroxyethylazol-Derivate der Formel (I) sowie deren Säureadditions-Salze und Metallsalz-Komplexe sehr gute fungizide und pflanzenwuchsregulierende Eigenschaften aufweisen.

Überraschenderweise besitzen die erfindungsgemäßen Wirkstoffe eine deutlich bessere fungizide und pflanzenwuchsregulierende Wirksamkeit als die konstitutionell ähnlichsten Stoffe, die aus dem Stand der Technik bekannt sind.

Die erfindungsgemäßen Wirkstoffe enthalten ein asymmetrisch substituiertes Kohlenstoffatomen und können deshalb in den beiden optischen Isomerenformen anfallen. Die Erfindung betrifft sowohl die Isomerengemische als auch die einzelnen Isomeren.

Besonders bevorzugt sind diejenigen Stoffe de Formel (I), in denen R für gegebenenfalls einfach bis dreifach, gleichartig oder verschieden substituiertes Phenyl steht, wobei als Substituenten genannt seien: Fluor, Chlor, Brom, Methyl, Ethyl, n-Propyl, n-Butyl, tert.-Butyl, Methoxy, Ethoxy, Methylthio, Ethylthio, Trifluormethyl, Trifluormethoxy, Trifluormethylthio, Cyclopentyl, Cyclohexyl, Phenyl, Phenoxy, Methoximinomethyl und Methoximinoethyl. Die Substituenten X und Y stehen unabhängig voneinander insbesondere für Wasserstoff, Fluor und Chlor.

Bevorzugte erfindungsgemäße Verbindungen sind auch Additionsprodukte aus Säuren und denjenigen 2-Hydroxyethylazol-Derivaten der Formel (I), in denen R, X und Y die Bedeutungen haben, die bereits für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II, bis IV, Haupt- und der I, und II, sowie IV, bis VIII, Nebengruppe des Periodensystems der Elemente und denjenigen 2-Hydroxyethyl-azol-Derivaten de Formel (I), in denen R, X und Y die Bedeutungen haben, die bereits für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man 2-(1,1-Dimethyl-ethyl)-2-(3-phenoxy-propyl)-oxiran und 1,2,4-Triazol als Ausgangsstoffe, so kann der Verlauf des erfindungsgemäßen Verfahrens durch das folgende Formelschema wiedergegeben werden:

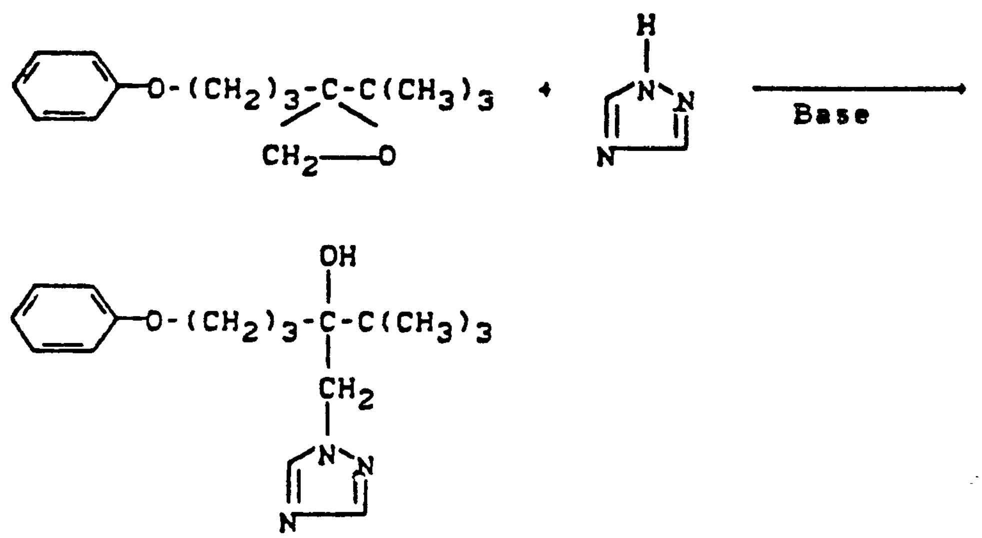


Dei bei der Durchführung des erfindungsgemäßen Verfahrens als Ausgangsstoffe benötigten Oxirane sind durch die Formel (II) allgemein definiert. In dieser Formel haben R, X und Y diejenigen Bedeutungen, die bereits bei der Beschreibung der erfindungsgemäßen Stoffe der Formel (I) für diese Substituenten genannt wurden.

Die Oxirane der Formel (II) sind neu. Sie lassen sich herstellen, indem man Phenoxypropylketone der Formel

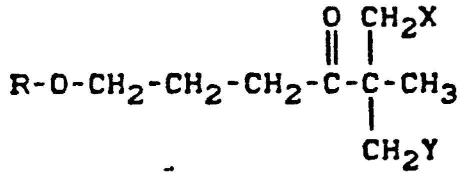

(IV)

in welcher

R, X und Y die oben angegebene Bedeutung haben,

mit Trimethylsulfoniumiodid oder Trimethylsulfoniummethosulfat in Gegenwart einer Base, wie z.B. Kalium-tert.-butylat, Kaliumhydroxid oder Natriumhydrid, sowie in Gegenwart eines Verdünnungsmittels, wie. z.B. Dimethylsulfoxid, Acetonitril oder Tetrahydrofuran, bei Temperaturen zwischen 0°C und 60°C umsetzt.

Die bei dem obigen Verfahren als Ausgangsstoffe benötigten Phenoxypropylketone sind durch die Formel (IV) definiert. In dieser Formel haben R, X und Y diejenigen Bedeutungen, die bereits im Zusammenhang mit der Beschreibung der erfindungsgemäßen Stoffe der Formel (I) für diese Substituenten genannt wurden.

Die Phenoxypropylketone der Formel (IV) sind teilweise bekannt (vgl. EP—OS 0 150 404). Sie lassen sich nach einem neuen Verfahren dadurch herstellen, daß man Säurehalogenide der Formel

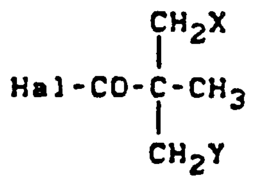

(V),

in welcher

X und Y die oben angegebene Bedeutung haben und

Hal für Chlor, Brom oder Iod steht,

mit Phenoxypropargyl-Verbindungen der Formel

$$R—O—CH_2—C{\equiv}C—H \qquad (VI)$$

in welcher

R die oben angegebene Bedeutung hat,

gegebenenfalls in Gegenwart eines Verdünnungsmittels und gegebenenfalls in Gegenwart eines Säurebindemittels sowie gegebenenfalls in Gegenwart eines Katalysators umsetzt und die dabei entstehenden phenoxy-substituierten Ketone der Formel

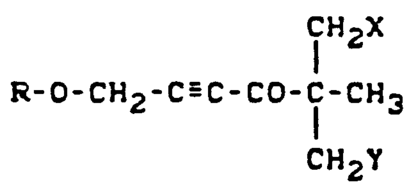

(VII)

in welcher

R, X und Y die oben angegebene Bedeutung haben,

mit Wasserstoff in Gegenwart eines Hydrierkatalysators sowie in Gegenwart eines Verdünnungsmittels umsetzt.

Überraschenderweise lassen sich die Phenoxypropylketone der Formel (IV) nach dem obigen Verfahren in höheren Ausbeuten herstellen als nach den bisher bekannten Verfahren. Darüberhinaus zeichnet sich das neue Verfahren gegenüber den entsprechenden vorbeschriebenen Methoden durch eine Reihe von Vorteilen aus. So sind die benötigten Ausgangsmaterialien einfach zu handhaben und auch in größeren Mengen zugänglich. Günstig ist auch, daß ein Arbeiten mit metallorganischen Verbindungen vermieden wird. Ferner ist keine intermediäre Oxidation von Carbinol-Gruppen erforderlich, die bei der Synthese von Endprodukten gegebenenfalls wieder reduziert werden.

Verwendet man bei dem obigen Verfahren Pivaloylchlorid und 4-Chlorphenyl-propargyl-ether als Ausgangsstoffe, Kupfer-(II)-chlorid als Katalysator und hydriert man das dabei entstehende 2,2-Dimethyl-6-(4-chlorphenoxy)-hex-4-in-3-on mit Wasserstoff in Gegenwart von Raney-Nickel, so kann der Reaktionsablauf durch das folgende Formelschema veranschaulicht werden:

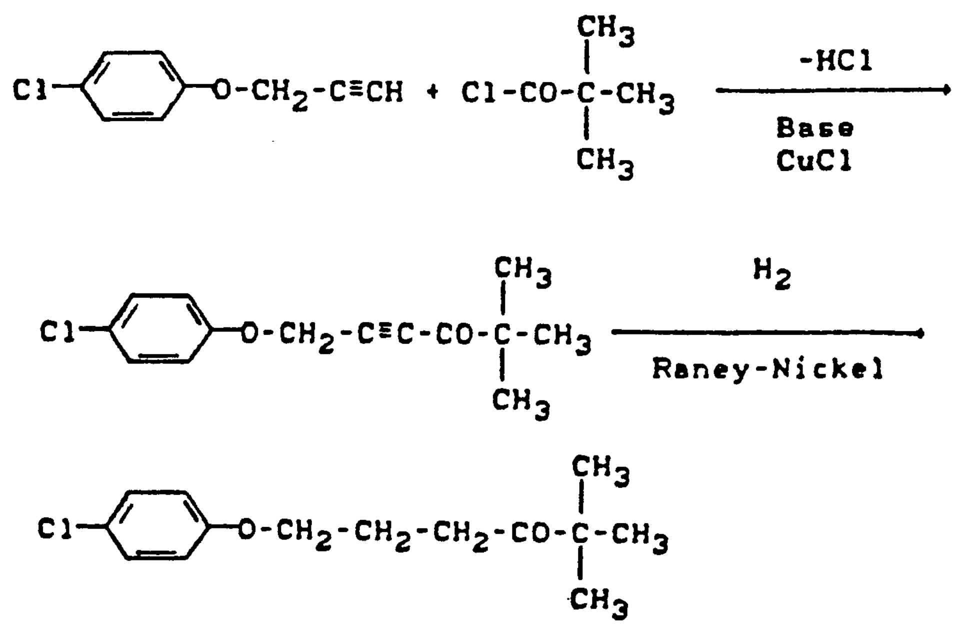


Die bei dem obigen Verfahren als Ausgangsstoffe benötigten Säurehalogenide sind durch die Formel (V) definiert. In dieser Formel haben X und Y diejenigen Bedeutungen, die bereits im Zusammenhang mit der Beschreibung der erfindungsgemäßen Stoffe der Formel (I) für diese Substituenten genannt wurden.

Die Säurehalogenide der Formel (V) sind bekannt oder können nach prinzipiell bekannten Verfahren hergestellt werden (vgl. US—PS 3 414 612, DE—OS 3 128 445 und EP—OS 0 049 416).

Die bei dem obigen Verfahren weiterhin als Ausgangsstoffe benötigten Phenoxypropargyl-Verbindungen sind durch die Formel (VI) definiert. In dieser Formel hat R diejenigen Bedeutungen, die bereits im Zusammenhang mit der Beschreibung der erfindungsgemäßen Stoffe der Formel (I) für diesen Substituenten genannt wurden.

Die Phenoxypropargyl-Verbindungen der Formel (VI) sind ebenfalls bekannt oder lassen sich nach im Prinzip bekannten Verfahren in einfacher Weise herstellen (vgl. Houben-Weyl "Methoden der organischen Chemie", Band V, 2a, S. 654).

Die bei dem obigen Verfahren als Zwischenprodukte entstehenden phenoxysubstituierten Ketone der Formel (VII) sind neu.

Als Verdünnungsmittel kommen bei der Durchführung der ersten Stufe des obigen Verfahrens inerte organische Solventien in Frage. Vorzugsweise verwendbar sind aromatische Kohlenwasserstoffe, wie Toluol, Xylol, Chlorbenzol, ferner Nitrile, wie Acetonitril, außerdem Ether, wie Diisobutyl-ether und Dioxan, und weiterhin auch Pyridin.

Als Säurebindemittel kommen bei der Durchführung der ersten Stufe des obigen Verfahrens vorzugsweise tertiäre Amine in Betracht. Besonders bevorzugt sind Triethylamin, N,N-Dimethylcyclohexylamin und N,N-Dimethylbenzylamin.

Die Reaktionstemperaturen können bei der Durchführung der ersten Stufe des obigen Verfahrens innerhalb eines größeren Bereiches variiert werden. Im allgemeinen arbeitet man bei Temperaturen zwischen 0°C und 150°C, vorzugsweise zwischen 20°C und 120°C.

Als Katalysatoren kommen bei der Durchführung der ersten Stufe des obigen Verfahrens vorzugsweise Kupfer-(I)-chlorid und Kupfer-(I)-bromid in Frage.

Bei der Durchführung der ersten Stufe des obigen Verfahrens setzt man die Verbindungen der Formeln (V) und (VI) in etwa äquimolaren Mengen ein. Es ist jedoch auch möglich, die eine oder andere Komponente in einem Überschuß zu verwenden. In vielen Fällen empfiehlt es sich, in Gegenwart eines Säurebindemittels und in Gegenwart einer katalytisch wirkenden Substanz zu arbeiten. Die Aufarbeitung und die Isolierung der phenoxy-substituierten Ketone der Formel (VII) erfolgen nach üblichen Methoden.

Bei der Durchführung der zweiten Stufe des obigen Verfahrens zur Herstellung von Phenoxypropylketonen der Formel (IV) arbeitet man in flüssiger Phase, vorzugsweise in Anwesenheit von Verdünnungsmitteln, unter Verwendung eines suspendierten, pulverförmigen Hydrierungskatalysators. Die Durchführung der Hydrierung kann diskontinuierlich (chargenweise) oder kontinuierlich als Sumpf- oder Rieselphasenhydrierung in bekannten Hydrierreaktoren, wie Autoklaven, Autoklavenkaskaden, Rohrreaktoren oder Umlaufreaktoren erfolgen. Die bevorzugte Arbeitsweise ist die diskontinuierliche Sumpfphasenhydrierung im Autoklaven bei erhöhtem Druck.

Als Verdünnungsmittel kommen bei der Durchführung der Hydrierung in der zweiten Stufe des obigen Verfahrens inerte organische Solventien in Frage. Vorzugsweise verwendbar sind Alkohole, wie Methanol, Ethanol, Isopropanol oder Ethylenglykol, ferner Ether, wie Diethylether, Diisopropylether,

Ethylenglykolmonomethylether, Ethylenglykoldimethylether, Dioxan oder Tetrahydrofuran; gesättigte Kohlenwasserstoffe, wie n-Heptan oder Cyclohexan; sowie Ester, wie Essigsäureethylester.

Für das obige Verfahren geeignete Hydrierkatalysatoren sind beispielsweise solche, die aus Metallen und/oder Verbindungen von Elementen der achten Nebengruppe des periodischen Systems der Elemente nach Mendelejew bestehen oder diese enthalten. Dabei sind die Metalle Ruthenium, Rhodium, Palladium, Platin, Kobalt und Nickel und deren Verbindungen bevorzugt. Bei den Metallverbindungen kann es sich beispielweise um Oxide, Hydroxide und/oder Oxihydrate handeln. Zusätzlich können die Metalle Kupfer, Vanadium, Molybdän, Chrom und/oder Mangan, sowie Verbindungen dieser Metalle zugegen sein.

Die Hydrierkatalysatoren können ausschließlich oder überwiegend aus wasserstoff-übertragenden Substanzen bestehen, diese können aber auch auf Trägermaterialien aufgebracht sein.

Als Trägermaterialien für die Wasserstoff-übertragenden Substanzen kommen beispielsweise in Frage: anorganische Materialien, wie Kieselgur, Kieselsäure, Aluminiumoxid, Alkali- und Erdalkalisilikate, Aluminiumsilikate, Montmorillonit, Zeolithe, Spinelle, Dolomit, Kaolin, Magnesiumsilikate, Zirkonoxid, Zinkoxid, Calciumcarbonat, Siliciumcarbid, Aluminiumphosphat, Borphosphat, Asbest, Aktivkohle oder Bariumsulfat, aber auch organische Materialien, beispielsweise natürlich vorkommende oder synthetische Verbindungen mit hohen Molekulargewichten, wie Seide, Polyamide, Polystyrole, Zellstoff oder Polyurethane, Bevorzugt sind anorganische Trägermaterialien in Pulverform.

Derartige Trägerkatalysatoren können im allgemeinen 0,5 bis 50 Gew.-%, vorzugsweise 1 bis 10 Gew.-% der Wasserstoff-überträgenden Substanz, bezogen auf die Gesamtmasse des Trägerkatalysators, enthalten. Die Wasserstoff-übertragende Substanz kann dabei homogen im Trägermaterial verteilt sein, bevorzugt sind jedoch Katalysatoren, in deren äußerer Schicht oder auf deren Oberfläche die Wasserstoff-übertragende Substanz abgelagert ist. Die Herstellung und die Formgebung der Katalysatoren, die im obigen Verfahren Verwendung finden können, kann in bekannter Weise erfolgen (siehe beispielsweise Houben-Weyl, Methoden der organischen Chemie, Band IV, Ic, Teil I, S. 16 bis 26, Georg Thieme Verlag, Stuttgart 1980).

Bevorzugte Trägerkatalysatoren sind Ruthenium auf Kohle, Ruthenium auf Aluminiumoxid, Rhodium auf Kohle, Rhodium auf Aluminiumoxid, Palladium auf Calciumcarbonat, Palladium auf Bariumsulfat, Palladium auf Kieselsäure, Platin auf Kohle und Platin auf Aluminiumoxid, Nickel auf Kieselgur, Nickel auf Aluminiumoxid sowie Nickel und Palladium auf Aluminiumoxid.

Bevorzugte Hydrierkatalysatoren, die ausschließlich oder überwiegend aus Wasserstoff-übertragender Substanz bestehen, sind beispielsweise oxidische Katalysatoren, wie Palladiumoxid, Platinoxid, Rutheniumoxid und/oder Rhodiumoxid/Platinoxid nach Nishimura, ferner durch Reduktion von entsprechenden Metallsalzen oder Metallsalzgemischen mit Alkalihydriden, Alkaliboranaten, Metallalkylen, Hydrazin, Formaldehyd, Wasserstoff oder elektropositiven Metallen herstellbare Schwarzkatalysatoren, wie Palladium/Schwarz, Platin/Schwarz und Rhodium/Schwarz; sowie Skelettkatalysatoren vom Raney-Typ, wie Raney-Nickel, Raney-Kobalt, Raney-Nickel-Kobalt, Raney-Nickel-Eisen, Raney-Nickel-Kupfer, Raney-Nickel-Eisen-Chrom, Raney-Nickel-Palladium und Raney-Nickel-Eisen-Vanadium.

Katalysatoren, die Nickel und/oder Palladium enthalten oder daraus bestehen, sind besonders bevorzugt.

Die Hydrierkatalysatoren werden bei der Durchführung der zweiten Stufe des obigen Verfahrens in einer solchen Menge eingesetzt, daß 0,05 bis 2,5, vorzugsweise 0,1 bis 1 Gew.-% Wasserstoff-übertragende Substanz bezogen auf das Gesamtgewicht des Reaktionsgemischs vorliegen.

Zur Durchführung des obigen Verfahrens können auch Gemische aus zwei oder mehreren der genannten Hydrierkatalysatoren verwendet werden.

Die katalytische Aktivität der Hydrierkatalysatoren bleibt bei der Durchführung des obigen Verfahrens im Allgemeinen weitgehend erhalten, so daß diese bei diskontinuierlicher Arbeitsweise wiederholt eingesetzt werden können und bei kontinuierlicher Arbeitsweise längere Zeit in Gebrauch bleiben können.

Die Reaktionstemperaturen können bei der Durchführung der Hydrierung in der zweiten Stufe des obigen Verfahrens in einem größeren Bereich variiert werden. Im allgemeinen arbeitet man bei Temperaturen zwischen 0°C und 150°C, vorzugsweise zwischen 20°C und 60°C.

Die Hydrierungen werden bei dem obigen Verfahren vorzugsweise bei erhöhtem Druck durchgeführt. Im allgemeinen arbeitet man bei Drucken zwischen 1 und 150 bar, vorzugsweise zwischen 20 und 80 bar.

Die für die obige Hydrierung erforderliche Reaktionszeit ist abhängig von der Reaktionstemperatur, dem Wasserstoffpartialdruck, der Intensität der Durchmischung des Reaktionsgemisches und von der Aktivität und Konzentration des Hydrierkatalysators. Im allgemeinen liegt die erforderliche Reaktionszeit im Bereich von 15 Minuten bis zu mehreren Stunden.

Die Hydrierung in der zweiten Stufe des obigen Verfahrens kann beispielsweise in der einfachsten Ausführungsform diskontinuierlich in folgender Weise durchgeführt werden: Ein mit einer Rühr- oder Mischeinrichtung versehener, temperierbarer Autoklav wird in geeigneter Weise mit einem phenoxysubstituierten Keton der Formel (VII), dem Hydrierkatalysator und dem Verdünnungsmittel beschickt. Nachdem der Autoklav entlüftet und sodann Wasserstoff bis zu dem gewünschten Druck aufgedrückt worden ist, wird das Gemisch unter intensiver Durchmischung auf die gewählte Reaktionstemperatur enhitzt. Der Reaktionsverlauf läßt sich leicht durch Messung des Wasserstoffverbräuches, der durch weitere Wasserstoffzufuhr ausgeglichen wird, verfolgen. Die

Hydrierung ist beendet, wenn kein Wasserstoff mehr verbraucht wird und die verbrauchte Wasserstoffmenge etwa der theoretisch erforderlichen Wasserstoffmenge entspricht.

Nach beendeter Hydrierung wird das Reaktionsgemisch abgekühlt, entspannt und in bekannter Weise, beispielsweise durch Abfiltrieren des Katalysators und Destillieren des Verdünnungsmittels, aufgearbeitet.

Die Phenoxypropylketone der Formel (IV) eignen sich nicht nur als Zwischenprodukte zur Herstellung der erfindungsgemäßen Stoffe der Formel (I), sondern können außerdem auch zur Synthese von anderen Stoffen mit fungiziden und pflanzenwuchsregulierenden Eigenschaften verwendet werden (vgl. EP—OS 0 150 404).

Die Oxirane der Formel (II) können bei dem erfindungsgemäßen Verfahren zur Herstellung von 2-Hydroxyethyl-azol-Derivaten der Formel (I) gegebenenfalls ohne Isolierung direkt weiter umgesetzt werden.

Als Verdünnungsmittel kommen für das erfindungsgemäße Verfahren zur Herstellung von 2-Hydroxyethyl-azol-Derivaten der Formel (I) alle inerten organischen Solventien in Frage. Vorzugsweise verwendbar sind Alkohole, wie Ethanol und Methoxyethanol; Ketone, wie z.B. 2-Butanon; Nitrile, wie z.B. Acetonitril; Ester, wie z.B. Essigester; Ether, wie z.B. Dioxan; aromatische Kohlenwasserstoffe, wie z.B. Benzol und Toluol; oder Amide, wie z.B. Dimethylformamid.

Als Basen kommen für die erfindungsgemäße Umsetzung alle überlichweise verwendbaren anorganischen und organischen Basen in Betracht. Hierzu gehören vorzugsweise Alkalicarbonate, wie z.B. Natrium- und Kaliumcarbonat; Alkalihydroxide, wie z.B. Natriumhydroxid; Alkalialkoholate, wie z.B. Natrium- und Kalium-methylat und -ethylat; Alkalihydride, wie z.B. Natriumhydride; sowie niedere tertiäre Alkylamine, Cycloalkylamine und Aralkylamine, wie insbesondere Triethylamin.

Die Reaktionstemperaturen können bei der Durchführung des erfindungsgemäßen Verfahrens in einem größeren Bereich variiert werden. Im allgemeinen arbeitet man bei Temperaturen zwischen 0 und 200°C, vorzugsweise zwischen 60 und 150°C.

Bei der Durchführung des erfindungsgemäßen Verfahrens setzt man auf 1 Mol Oxiran der Formel (II) vorzugsweise 1 bis 2 Mol 1,2,4-Triazol und gegebenenfalls 1 bis 2 Mol Base ein. Die Isolierung der Endprodukte erfolgt in allgemein üblicher Weise.

Die nach dem erfindungsgemäßen Verfahren erhältlichen Verbindungen der Formel (I) können in Säureadditions-Salze bzw. Metallsalze-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen in Frage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol, und Hinzufügen zu Verbindungen der allgemeinen Formel (I), Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuligenea;
Podosphaera-Arten, wie Podosphaera leucotricha;
Venturia-Arten, wie Venturia inaequalis;
Pyrenophora-Arten, wie Pyrenophora teres oder P. graminea;
(Konidienform; Drechslera, Syn: Helminthosporium); Cochliobolus-Arten, wie Cochliobolus sativus;
(Konidienform: Drechslera, Syn: Helminthosporium);
Uromyces-Arten, wie Uromyces appendiculatus;

Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Außerdem besitzen die erfindungsgemäßen Wirkstoffe auch pflanzenwuchsregulierende Eigenschaften.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß eine Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmungs des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen, auszubringen, um den Ertrag zu steigern, ohne daß die Gefahr besteht, daß das Getreide lagert.

Eine Hemmung des vegetativen Wachstums ermöglicht, bei vielen Kulturpflanzen eine dichtere Ampflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Verängerung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflüßt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein

kann. Andererseits ists es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann eine vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, dab z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z.B. Ananas oder Zierpflanzen in Gärtnereien, zu einem Zeitpunkt keimen, austreiben oder blühen, an dem sie normalerweise hierzu keine Berreitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Läsungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittle kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Die Wirkstoffe können als solche, in Form ihrer Formulierungen oder den daraus bereiteten Anwendungsformen wie bebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht, in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Herstellungsbeispiele

Biespiel 1

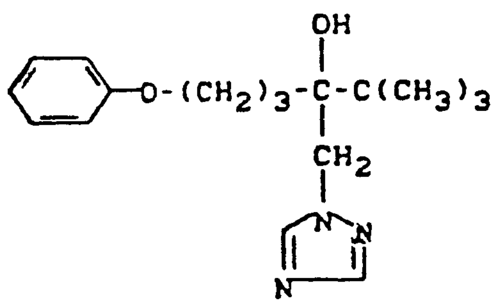

(I-1)

In eine Suspension von 11,3 g (0,082 Mol) Kaliumcarbonat in 50 ml Dimethylformamid werden 5,6 g (0,082 Mol) 1,2,4-Triazol gegeben. Anschließend tropft man unter Rühren 8,8 g (0,038 Mol) 2-(1,1-Dimethylethyl)-2-(3-phenoxypropyl)-oxiran in die Mischung und erhitzt 12 Stunden auf 130 bis 140°C. Nach dem Abkühlen auf Raumtemperatur wird das Reaktionsgemisch filtriert. Das Filtrat wird unter vermindertem Druck eingeegt, und der verbleibende Rückstand wird in Methylenchlorid gelöst. Die organische Phase wird dreimal mit Wasser gewaschen und nach dem Trocknen eingeengt. Der Rückstand wird aus n-Hexan umkristallisiert. Man erhält auf diese Weise 8,1 g (70,3 % der Theorie) an 2,2-Dimethyl-6-phenoxy-3-(1,2,4-triazol-1-yl-methyl)-hexan-3-ol vom Schemlzpunkt 106°C.

Herstellung von Ausgangsprodukten:

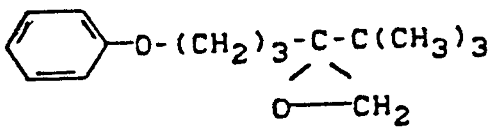

(II-1)

9 g (41 mMol) 2,2-Dimethyl-6-phenoxy-hexan-3-on werden unter Rühren bei Raumtemperatur in ein Gemisch aus 5,5 g (49 mMol) Kalium-tert.-butylat in 40 ml Tetrahydrofuran getropft, wobei sich eine klare gelbliche Lösung bildet. Anschließend fügt man 12,5 g (61,5 mMol) Trimethylsulfoniumiodid hinzu und rührt 12 Stunden bei 20 bis 25°C. Danach wird das Reaktionsgemisch filtriert und das Filtrat unter vermindertem Druck eingeengt. Der verbleibende Rückstande wird in Methylenchlorid aufgenommen und die entstehende Lösung wird dreimal mit Wasser gewaschen. Nach dem Trocknen der organischen Phase

wird das Lösungsmittel abgezogen. Man erhält auf diese Weise 7,8 g (81 % der Theorie) an 2-(1,1-Dimethyl-ethyl)-2-(3-phenoxy-propyl)-oxiran in Form einer Flüssigkeit mit dem Brechungsindex $n_D^{20}$ = 1,503.

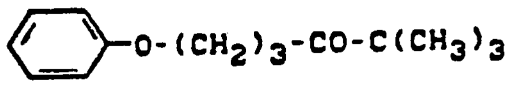
(IV-1)

20 g (0,0926 Mol) 2,2-Dimethyl-6-phenoxy-hex-4-in-3-on werden in 180 ml Methanol gelöst und mit 5 g Raney-Nickel versetzt. Man hydriert bei 37°C mit Wasserstoff unter einem Druck von 50 bis 60 bar. Die Hydrierung ist nach 40 Minuten beendet. Das Reaktionsgemisch wird filtriert, und das Filtrat wird durch Abziehen des Lösungsmittels unter vermindertem Druck eingeengt. Man erhält auf diese Weise 19,5 g (95,7 % der Theorie) an 2,2-Dimethyl-6-phenoxy-hexane-3-on.

$^1$H—NMR($CDCl_3$): s (9H) bei 1,1 ppm; m (2H) bei 2,05 ppm, t (2H) bei 2,7 ppm, t (2H) bei 3,95 ppm und m (5H) bei 6,85—7,3 ppm.

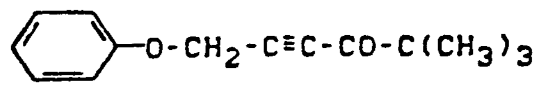
(VII-1)

In ein Gemisch aus 10,1 g (0,1 Mol) Triethylamin und 1,43 g Kupfer-(I)-bromid in 60 ml Toluol werden unter Stickstoffatmosphäre und unter Rühren bei 20°C 13,2 g (0,1 Mol) Phenyl-propargylether eingetropft. Es wird 30 Minuten bei 20°C nachgerührt. Danach werden innerhalb von 15 Minuten 12 g (0,1 Mol) Pivalinsäurechlorid zugetropft. Man erhitzt das Reaktionsgemisch auf 80°C und rührt 10 Stunden bei dieser Temperatur. Nach dem Abkühlen auf Raumtemperatur wird das Reaktionsgemisch zunächst mit verdünnter wäßriger Salzäure und dann mit Wasser gewaschen. Die organische Phase wird durch Abziehen des Lösungsmittels eingeengt, und der verbleibende Rückstand wird im Hochvakuum destilliert. Man erhält auf diese Weise 16 g (75 % der Theorie) an 2,2-Dimethyl-6-phenoxy-hex-4-in-3-on in Form einer Flüssigkeit. Kp = 125°C/0,05 mbar (Kugelrohr).

Nach der in Beispiel 1 angegebenen Methode werden auch die in den folgenden Beispielen aufgeführten Verbindungen erhalten.

Beispiel 2

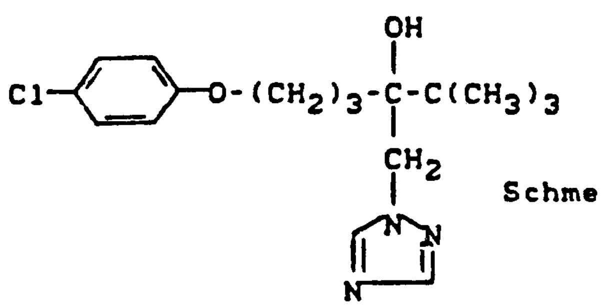
Schmelzpunkt: 87° C (I-2)

Beispiel 3

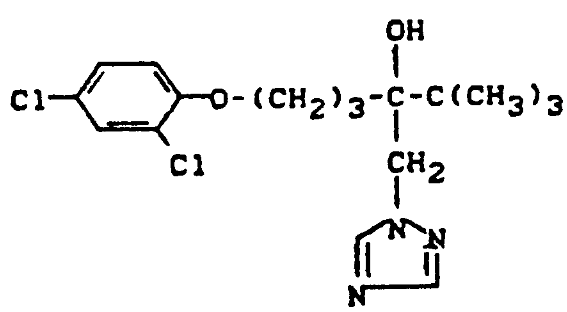
Öl (I-3)

Beispiel 4

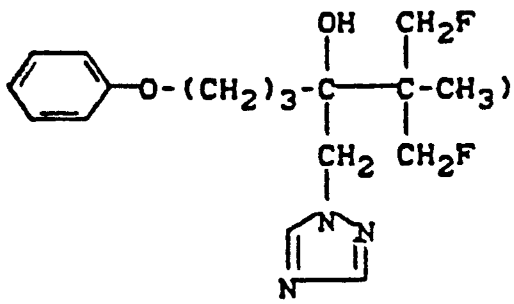

Nach der im Beispiel 1 angegebenen Methode werden auch die in den folgenden Beispielen aufgeführten Ausgangsprodukte hergestellt.

Beispiel 5

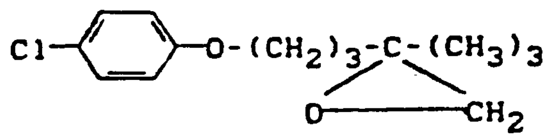

(II-2)

$n_D^{20} = 1,515$

Beispiel 6

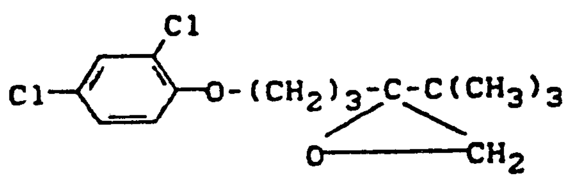

(II-3)

$n_D^{20} = 1,529$

Beispiel 7

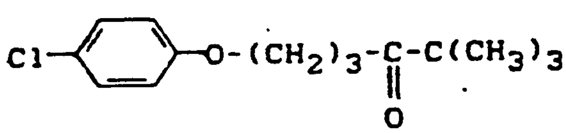

(IV-2)

$n_D^{20} = 1,509$

Beispiel 8

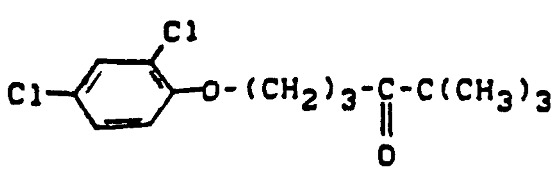

(IV-3)

$n_D^{20} = 1,512$

Beispiel 9

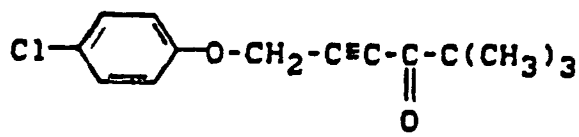

(VII-2)

$n_D^{20}$ = 1,519

Beispiel 10

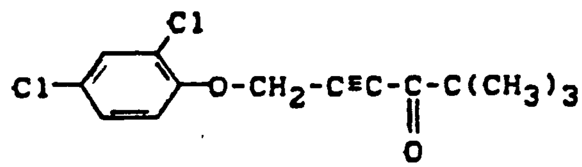

(VII-3)

$n_D^{20}$ = 1,535

In den folgenden Beispielen wurde die Verbindung der nachstehend angegebenen Formel jeweils als Vergleichskomponente eingesetzt:

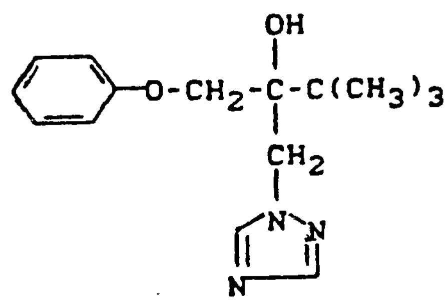

= (A)

(Bekannt aus EP—OS 0 040 345)

Beispiel A

Wuchshemmung bei Baumwolle

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 2 Wochen wird der Zuwachs der Pflanzen gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

In diesem Test zeigt der erfindungsgemäße Wirkstoff der Formel (I—1) eine wesentlich bessere Wirkung als die Vergleichssubstanz (A).

Beispiel B

Wachstum bei Zuckerrüben

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit den Wirkstoffzubereitungen besprüht. Nach 14 Tagen wird der Zuwachs der Pflanzen gemessen und das Wachstum in Prozent des Zuwachses der Kontrollpflanzen berechnet. Es bedeuten 100 % Wachstum ein Wachstum entsprechend dem der Kontrollpflanzen, Werte

über 100 % eine Steigerung des Wachstums gegenüber den Kontrollpflanzen und 0 % den Stillstand des Wachstums.

In diesem Test zeigt der erfindungsgemäße Wirkstoff der Formel (I—1) eine bessere Wirkung als die Vergleichssubstanz (A).

Beispiel C

Pyricularia-Test (Reis)/protektiv

Lösungsmittel: 12,5 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

In diesem Test zeigt der erfindungsgemäße Wirkstoff der Formel (I—1) eine wesentlich besser Wirkung als die Vergleichssubstanz (A).

Beispiel D

Puccinia-Test (Weizen)/protektiv

Lösungsmittel: 100 Gewichtsteile Dimethylformamid

Emulgator: 0,25 Gewichtsteile Alcylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita mit in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt, um die Entwicklung von Rostpusteln zu begünstigen.

Die Auswertung erfolgt 10 Tage nach der Inokulation.

In diesem Test zeigt der erfindungsgemäße Wirkstoff der Formel (I—1) eine wesentlich bessere Wirksamkeit als die Vergleichssubstanz (A).

Beispiel E

Venturia-Test (Apfel)/protektiv

Lösungsmittel: 4,7 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

In diesem Test zeigt der erfindungsgemäße Wirkstoff der Formel (I—1) eine wesentlich bessere Wirksamkeit als die Vergleichssubstanz (A).

**Patentansprüche**

1. 2-Hydroxyethyl-azol-Derivate der Formel

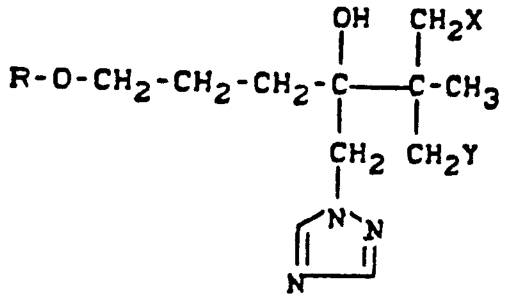

(I)

in welcher

R für gegebenenfalls einfach bis dreifach, gleichartig oder verschieden substituiertes Phenyl steht, wobei als Substituenten in Frage kommen; Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Cycloalkyl mit 3 bis 7 Kohlenstoffatomen, Phenyl, Phenoxy und Alkoximinoalkyl mit 1 bis 4 Kohlenstoffatomen in der Alkoxygruppe und 1 bis 4 Kohlenstoffatomen in der Alkylgruppe,

X für Wasserstoff, Fluor, Chlor oder Brom steht und

Y für Wasserstoff, Fluor, Chlor oder Brom steht,

sowie deren Säureadditions-Salze und Metallsalz-Komplexe.

2. Verfahren zur Herstellung von 2-Hydroxyethyl-azol-Derivaten der Formel

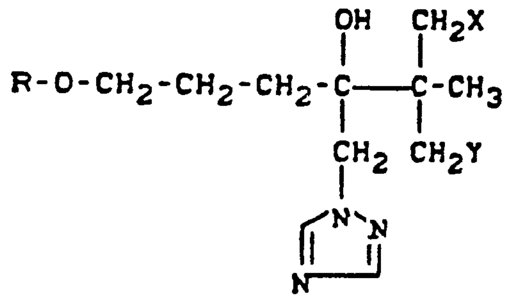

(I)

in welcher

R für gegebenenfalls einfach bis dreifach, gleichartig oder verschieden substituiertes Phenyl steht, wobei als Substituenten in Frage kommen; Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Cycloalkyl mit 3 bis 7 Kohlenstoffatomen, Phenyl, Phenoxy und Alkoximinoalkyl mit 1 bis 4 Kohlenstoffatomen in der Alkoxygruppe und 1 bis 4 Kohlenstoffatomen in der Alkylgruppe,

X für Wasserstoff, Fluor, Chlor oder Brom steht und

Y für Wasserstoff, Fluor, Chlor oder Brom steht,

sowie deren Säureadditions-Salzen und Metallsalz-Komplexen, dadurch gekennzeichnet, daß man Oxirane der Formel

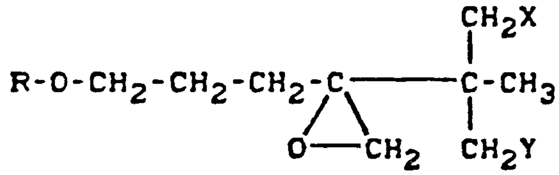

(II)

in welcher

R, X und Y die oben angegebene Bedeutung haben, mit 1,2,4-Triazol der Formel

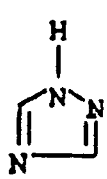

(III)

in Gegenwart eines Verdünnungsmittels und gegebenenfalls in Gegenwart einer Base umsetzt und

gegebenenfalls anschließend an die so erhaltenen Verbindungen der Formel (I) eine Säure oder ein Metallsalze addiert.

3. Fungizide und pflanzenwuchsregulierende Mittel, gekennzeichnet durch einen Gehalt an mindestens einem 2-Hydroxyethyl-azol-Derivat de Formel (I) gemäß Anspruch 1 bzw. an einem Säureadditions-Salz oder Metallsalz-Komplex eines 2-Hydroxyethyl-azol-Derivates der Formel (I).

4. Verwendung von 2-Hydroxyethyl-azol-Derivaten der Formel (I) gemäß Anspruch 1 bzw. von deren Säureadditions-Salzen und Metallsalz-Komplexen zur Bekämpfung von Pilzen sowie zur Regulierung des Pflanzenwachstums.

5. Verfahren zur Herstellung von fungiziden und pflanzenwuchsregulierenden Mitteln, dadurch gekennzeichnet, daß man 2-Hydroxyethyl-azol-Derivate der Formel (I) gemäß Anspruch 1 bzw. deren Säureadditionssalze oder Metallsalz-Komplexe mit Streckmitteln und/oder oberflächenaktiven Stoffen vermischt.

6. Oxirane der Formel

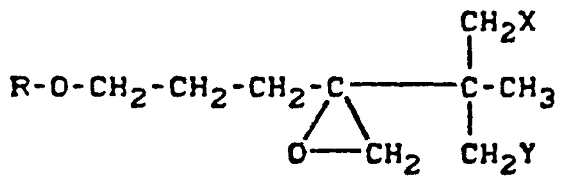

(II)

in welcher

R für gegebenenfalls einfach bis dreifach, gleichartig oder verschieden substituiertes Phenyl steht, wobei als Substituenten in Frage kommen; Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Cycloalkyl mit 3 bis 7 Kohlenstoffatomen, Phenyl, Phenoxy und Alkoximinoalkyl mit 1 bis 4 Kohlenstoffatomen in der Alkoxygruppe und 1 bis 4 Kohlenstoffatomen in der Alkylgruppe,

X für Wasserstoff, Fluor, Chlor oder Brom steht und

Y für Wasserstoff, Fluor, Chlor oder Brom steht.

7. Verfahren zur Herstellung von Oxiranen der Formel

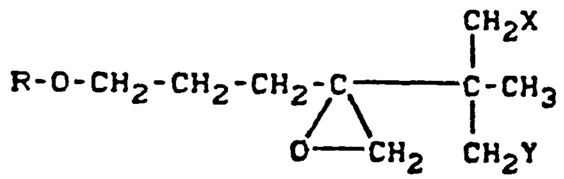

(II)

in welcher

R für gegebenenfalls einfach bis dreifach, gleichartig oder verschieden substituiertes Phenyl steht, wobei als Substituenten in Frage kommen; Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Cycloalkyl mit 3 bis 7 Kohlenstoffatomen, Phenyl, Phenoxy und Alkoximinoalkyl mit 1 bis 4 Kohlenstoffatomen in der Alkoxygruppe und 1 bis 4 Kohlenstoffatomen in der Alkylgruppe,

X für Wasserstoff, Fluor, Chlor oder Brom steht und

Y für Wasserstoff, Fluor, Chlor oder Brom steht, dadurch gekennzeichnet, daß man Phenoxypropylketone der Formel

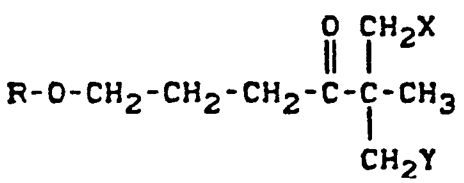

(IV)

in welcher

R, X und Y die oben angegebene Bedeutung haben, mit Trimethylsulfoniumiodid oder Trimethylsulfoniummethosulfat in Gegenwart einer Base sowie in Gegenwart eines Verdünnungsmittels umsetzt.

8. Phenoxy-substituierte Ketone der Formel

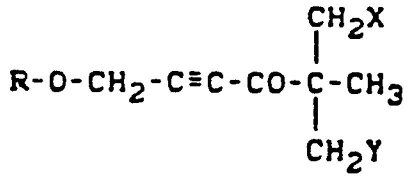

(VII)

in welcher

R für gegebenenfalls einfach bis dreifach, gleichartig oder verschieden substituiertes Phenyl steht, wobei als Substituenten in Frage kommen; Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Cycloalkyl mit 3 bis 7 Kohlenstoffatomen, Phenyl, Phenoxy und Alkoximinoalkyl mit 1 bis 4 Kohlenstoffatomen in der Alkoxygruppe und 1 bis 4 Kohlenstoffatomen in der Alkylgruppe,

X für Wasserstoff, Fluor, Chlor oder Brom steht und

Y für Wasserstoff, Fluor, Chlor oder Brom steht.

9. Verfahren zur Herstellung von Phenoxypropylketonen der Formel

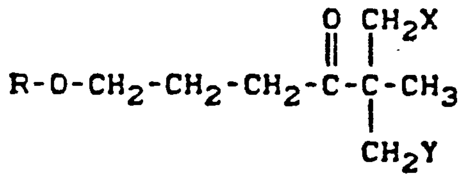

(IV)

in welcher

R für gegebenenfalls einfach bis dreifach, gleichartig oder verschieden substituiertes Phenyl steht, wobei als Substituenten in Frage kommen; Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Cycloalkyl mit 3 bis 7 Kohlenstoffatomen, Phenyl, Phenoxy und Alkoximinoalkyl mit 1 bis 4 Kohlenstoffatomen in der Alkoxygruppe und 1 bis 4 Kohlenstoffatomen in der Alkylgruppe,

X für Wasserstoff, Fluor, Chlor oder Brom steht und

Y für Wasserstoff, Fluor, Chlor oder Brom steht, dadurch gekennzeichnet, daß man Säurehalogenide der Formel

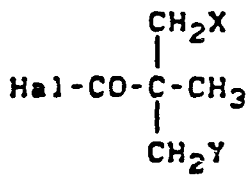

(V),

in welcher

X und Y die oben angegebene Bedeutung haben und

Hal für Chlor, Brom oder Iod steht,

mit Phenoxypropargyl-Verbindungen der Formel

$$R—O—CH_2—C\equiv C—H \qquad (VI)$$

in welcher

R die oben angegebene Bedeutung hat,

gegebenenfalls in Gegenwart eines Verdünnungsmittels und gegebenenfalls in Gegenwart eines Säurebindemittels sowie gegebenenfalls in Gegenwart eines Katalysators umsetzt und die dabei entstehenden phenoxy-substituierten Ketone der Formel

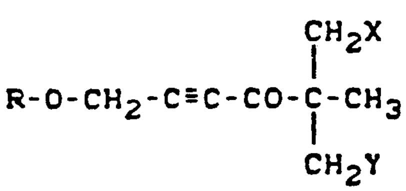

(VII)

in welcher

R, X und Y die oben angegebene Bedeutung haben,

mit Wasserstoff in Gegenwart eines Hydrierkatalysators sowie in Gegenwart eines Verdünnungsmittels umsetzt.

**Revendications**

1. Dérivés du 2-hydroxyéthylazole de formule

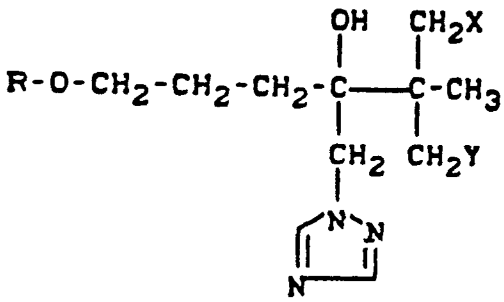

(I)

dans laquelle

R représente un groupe phényle éventuellement substitué une à trois fois, de façon identique ou différente, et l'on peut alors citer comme substituants: un halogène, un groupe alkyle ayant 1 à 4 atomes de carbone, un groupe alcoxy ayant 1 à 4 atomes de carbone, un groupe alkylthio ayant 1 à 4 atomes de carbone, un groupe halogénoalkyle ayant 1 ou 2 atomes de carbone et 1 à 5 atomes d'halogène, un groupe halogénoalcoxy ayant 1 ou 2 atomes de carbone et 1 à 5 atomes d'halogène, un groupe halogénoalkylthio ayant 1 ou 2 atomes de carbone et 1 à 5 atomes d'halogène, un groupe cycloalkyle ayant 3 à 7 atomes de carbone, un groupe phényle, un groupe phénoxy et un groupe alcoxy iminoalkyle ayant 1 à 4 atomes de carbone dans le groupe alcoxy et 1 à 4 atomes de carbone dans le groupe alkyle,

X représente un atome d'hydrogène, de fluor, de chlore ou de brome, et

Y représente un atome d'hydrogène, de fluor, de chlore ou de brome,

ainsi que leurs sels d'addition d'acides et leurs complexes avec des sels de métaux.

2. Procédé pour préparer des dérivés du 2-hydroxyéthylazole de formule

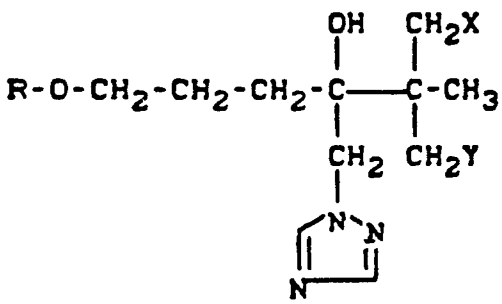

(I)

dans laquelle

R représente un groupe phényle éventuellement substitué une à trois fois, de façon identique ou différente, et l'on peut alors citer comme substituants: un halogène, un groupe alkyle ayant 1 à 4 atomes de carbone, un groupe alcoxy ayant 1 à 4 atomes de carbone, un groupe alkylthio ayant 1 à 4 atomes de carbone, un groupe halogénoalkyle ayant 1 ou 2 atomes de carbone et 1 à 5 atomes d'halogène, un groupe halogénoalcoxy ayant 1 ou 2 atomes de carbone et 1 à 5 atomes d'halogène, un groupe halogénoalkylthio ayant 1 ou 2 atomes de carbone et 1 à 5 atomes d'halogène, un groupe cycloalkyle ayant 3 à 7 atomes de carbone, un groupe phényle, un groupe phénoxy et un groupe alcoxy iminoalkyle ayant 1 à 4 atomes de carbone dans le groupe alcoxy et 1 à 4 atomes de carbone dans le groupe alkyle,

X représente un atome d'hydrogène, de fluor, de chlore ou de brome, et

Y représente un atome d'hydrogène, de fluor, de chlore ou de brome,

ainsi que leurs sels d'addition d'acide et leurs complexes avec des sels de métaux, procédé caractérisé en ce qu'on fait réagir des oxirannes de formule

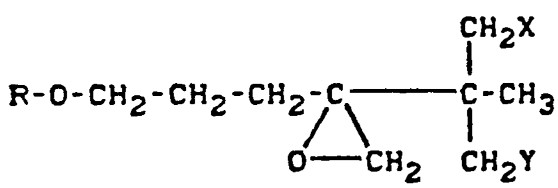

(II)

dans laquelle

R, X et Y ont le sens indiqué ci-dessus,

avec le 1,2,4-triazole de formule

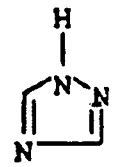

(III)

en présence d'un diluant et éventuellement en présence d'une base, et l'on fixe éventuellement ensuite, sur les composés de formule (I) ainsi obtenus, par addition un acide ou un sel de métal.

3. Produits fongicides et produits pouvant réguler la croissance de plantes, caractérisés en ce qu'ils contiennent au moins un dérivé de 2-hydroxyéthylazole de formule (I) selon la revendication 1 ou un sel d'addition d'acide ou un complexe avec un sel de métal d'un dérivé de 2-hydroxyéthylazole de formule (I).

4. Utilisation de dérivés du 2-hydroxyéthylazole de formule (I) selon la revendication 1 ou de leurs sels d'addition d'acide ou de leurs complexes avec des sels de mètaux pour combattre des champignons ainsi que pour réguler la croissance de plantes.

5. Procédé pour préparer des produits fongicides et des produits capables de réguler la croissance de plantes, procédé caractérisé en ce qu'on mélange des dérivés du 2-hydroxyéthylazole de formule (I) selon la revendication 1, ou leurs sels d'addition d'acide ou leurs complexes avec des sels de métaux, avec des agents d'allongement et/ou avec des substances tensioactives.

6. Oxirannes de formule

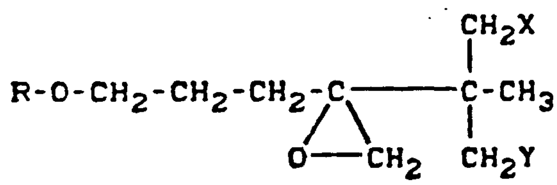

(II)

dans laquelle

R représente un groupe phényle éventuellement substitué une à trois fois, de façon identique ou différente, et l'on peut alors citer comme substituants: un halogène, un groupe alkyle ayant 1 à 4 atomes de carbone, un groupe alcoxy ayant 1 à 4 atomes de carbone, un groupe alkylthio ayant 1 à 4 atomes de carbone, un groupe halogénoalkyle ayant 1 ou 2 atomes de carbone et 1 à 5 atomes d'halogène, un groupe halogénoalcoxy ayant 1 ou 2 atomes de carbone et 1 à 5 atomes d'halogène, un groupe halogénoalkylthio ayant 1 ou 2 atomes de carbone et 1 à 5 atomes d'halogène, un groupe cycloalkyle ayant 3 à 7 atomes de carbone, un groupe phényle, un groupe phénoxy et un groupe alcoxy iminoalkyle ayant 1 à 4 atomes de carbone dans le groupe alcoxy et 1 à 4 atomes de carbone dans le groupe alkyle,

X représente un atome d'hydrogène, de fluor, de chlore ou de brome, et

Y représente un atome d'hydrogène, de fluor, de chlore ou de brome.

7. Procédé pour préparer des oxirannes de formule

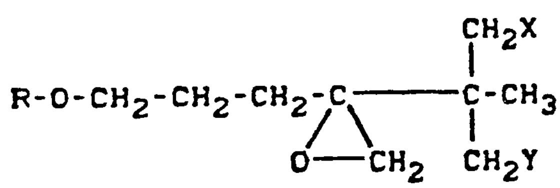

(II)

dans laquelle

R représente un groupe phényle éventuellement substitué une à trois fois, de façon identique ou différente, et l'on peut alors citer comme substituants: un halogène, un groupe alkyle ayant 1 à 4 atomes de carbone, un groupe alcoxy ayant 1 à 4 atomes de carbone, un groupe alkylthio ayant 1 à 4 atomes de carbone, un groupe halogénoalkyle ayant 1 ou 2 atomes de carbone et 1 à 5 atomes d'halogène, un groupe halogénoalcoxy ayant 1 ou 2 atomes de carbone et 1 à 5 atomes d'halogène, un groupe halogénoalkylthio ayant 1 ou 2 atomes de carbone et 1 à 5 atomes d'halogène, un groupe cycloalkyle ayant 3 à 7 atomes de carbone, un groupe phényle, un groupe phénoxy et un groupe alcoxy iminoalkyle ayant 1 à 4 atomes de carbone dans le groupe alcoxy et 1 à 4 atomes de carbone dans le groupe alkyle,

X représente un atome d'hydrogène, de fluor, de chlore ou de brome, et

Y représente un atome d'hydrogène, de fluor, de chlore ou de brome

procédé caractérisé en ce qu'on fait réagir des phénoxypropylcétones de formule

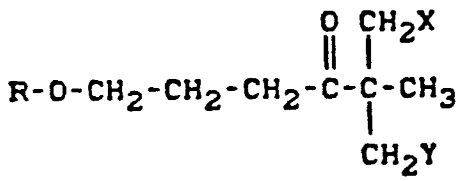

(IV)

dans laquelle

R, X et Y ont le sens précité,

avec l'iodure de triméthylsulfonium ou avec le méthosulfate de triméthylsulfonium, en opérant en présence d'une base ainsi qu'en présence d'un diluant.

8. Phénoxycétones de formule

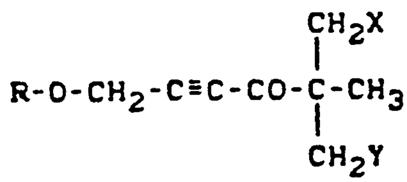

(VII)

dans laquelle

R représente un groupe phényle éventuellement substitué une à trois fois, de façon identique ou différente, et l'on peut alors citer comme substituants: un halogène, un groupe alkyle ayant 1 à 4 atomes de carbone, un groupe alcoxy ayant 1 à 4 atomes de carbone, un groupe alkylthio ayant 1 à 4 atomes de carbone, un groupe halogénoalkyle ayant 1 ou 2 atomes de carbone et 1 à 5 atomes d'halogène, un groupe halogénoalcoxy ayant 1 ou 2 atomes de carbone et 1 à 5 atomes d'halogène, un groupe halogénoalkylthio ayant 1 ou 2 atomes de carbone et 1 à 5 atomes d'halogène, un groupe cycloalkyle ayant 3 à 7 atomes de carbone, un groupe phényle, un groupe phénoxy et un groupe alcoxy iminoalkyle ayant 1 à 4 atomes de carbone dans le groupe alcoxy et 1 à 4 atomes de carbone dans le groupe alkyle,

X représente un atome d'hydrogène, de fluor, de chlore ou de brome, et

Y représente un atome d'hydrogène, de fluor, de chlore ou de brome.

9. Procédé pour préparer des phénoxypropylcétones de formule

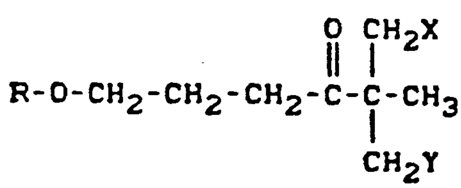

(IV)

dans laquelle

R représente un groupe phényle éventuellement substitué une à trois fois, de façon identique ou différente, et l'on peut alors citer comme substituants: un halogène, un groupe alkyle ayant 1 à 4 atomes de carbone, un groupe alcoxy ayant 1 à 4 atomes de carbone, un groupe alkylthio ayant 1 à 4 atomes de carbone, un groupe halogénoalkyle ayant 1 ou 2 atomes de carbone et 1 à 5 atomes d'halogène, un groupe halogénoalcoxy ayant 1 ou 2 atomes de carbone et 1 à 5 atomes d'halogène, un groupe halogénoalkylthio ayant 1 ou 2 atomes de carbone et 1 à 5 atomes d'halogène, un groupe cycloalkyle ayant 3 à 7 atomes de carbone, un groupe phényle, un groupe phénoxy et un groupe alcoxy iminoalkyle ayant 1 à 4 atomes de carbone dans le groupe alcoxy et 1 à 4 atomes de carbone dans le groupe alkyle,

X représente un atome d'hydrogène, de fluor, de chlore ou de brome, et

Y représente un atome d'hydrogène, de fluor, de chlore ou de brome,

procédé caractérisé en ce qu'on fait réagir des halogénures d'acides de formule

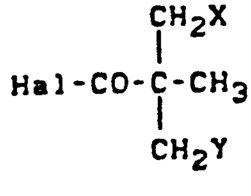

(V),

dans laquelle

X et Y ont le sens précité, et

Hal représente un atome de chlore, de brome ou d'iode, avec des composés phénoxypropargyliques de formule

$$R—O—CH_2—C≡C—H \quad (VI)$$

dans laquelle

R a le sens précité,

éventuellement en présence d'un diluant et éventuellement en présence d'un agent de fixation des acides ainsi qu'éventuellement en présence d'un catalyseur, et l'on fait réagir les phénoxycétones résultantes, de formule

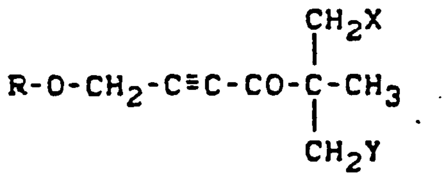

(VII)

dans laquelle

R, X et Y ont le sens précité,

avec l'hydrogène en présence d'un catalyseur d'hydrogénation ainsi qu'en présence d'un diluant.

**Claims**

1. 2-Hydroxyethyl-azole derivatives of the formula

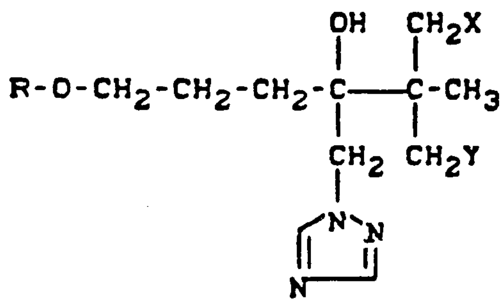

(I)

in which

R represents phenyl which is optionally monosubstituted, disubstituted or trisubstituted by identical or different substituents, possible substituents being: halogen, alkyl with 1 to 4 carbon atoms, alkoxy with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, cycloalkyl with 3 to 7 carbon atoms, phenyl, phenoxy and alkoximinoalkyl with 1 to 4 carbon atoms in the alkoxy group and 1 to 4 carbon atoms in the alkyl group,

X represents hydrogen, fluorine, chlorine or bromine and

Y represents hydrogen, fluorine, chlorine or bromine, and acid addition salts and metal salt complexes thereof.

2. Process for the preparation of 2-hydroxyethyl-azole derivatives of the formula

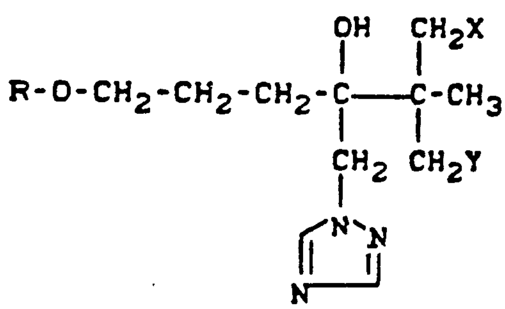

(I)

in which

R represents phenyl which is optionally monosubstituted, disubstituted or trisubstituted by identical or different substituents, possible substituents being: halogen, alkyl with 1 to 4 carbon atoms, alkoxy with 1 to

4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, cycloalkyl with 3 to 7 carbon atoms, phenyl, phenoxy and alkoximinoalkyl with 1 to 4 carbon atoms in the alkoxy group and 1 to 4 carbon atoms in the alkyl group,

X represents hydrogen, fluorine, chlorine or bromine and

Y represents hydrogen, fluorine, chlorine or bromine,

and of acid addition salts and metal salt complexes thereof, characterized in that oxiranes of the formula

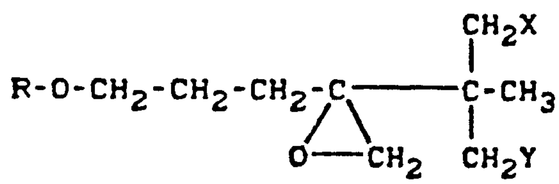

(II)

in which

R, X and Y have the abovementioned meaning, are reacted with 1,2,4-triazole of the formula

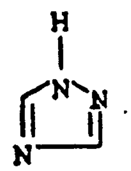

(III)

in the presence of a diluent and if appropriate in the presence of a base, and if appropriate an acid or a metal salt is subsequently added onto the compounds of the formula (I) thus obtained.

3. Fungicidal and plant growth-regulating agents, characterized in that they contain at least one 2-hydroxyethyl-azole derivative of the formula (I) according to Claim 1 or an acid additional salt or metal salt complex of a 2-hydroxyethyl-azole derivative of the formula (I).

4. Use of 2-hydroxyethyl-azole derivatives of the formula (I) according to Claim 1 or of acid addition salts and metal salt complexes thereof for combating fungi and for regulating plant growth.

5. Process for the preparation of fungicidal and plant growth-regulating agents, characterized in that 2-hydroxyethyl-azole derivatives of the formula (I) according to Claim 1 or acid addition salts or metal salt complexes thereof are mixed with extenders and/or surface-active substances.

6. Oxiranes of the formula

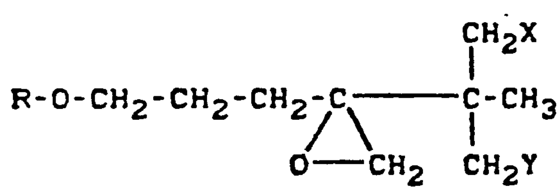

(II)

in which

R represents phenyl which is optionally monosubstituted, disubstituted or trisubstituted by identical or different substituents, possible substituents being: halogen, alkyl with 1 to 4 carbon atoms, alkoxy with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, cycloalkyl with 3 to 7 carbon atoms, phenyl, phenoxy and alkoximinoalkyl with 1 to 4 carbon atoms in the alkoxy group and 1 to 4 carbon atoms in the alkyl group,

X represents hydrogen, fluorine, chlorine or bromine and

Y represents hydrogen, fluorine, chlorine or bromine.

7. Process for the preparation of oxiranes of the formula

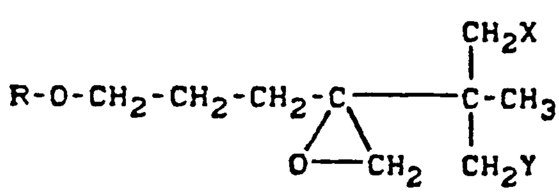

(II)

in which

R represents phenyl which is optionally monosubstituted, disubstituted or trisubstituted by identical or

different substituents, possible substituents being: halogen, alkyl with 1 to 4 carbon atoms, alkoxy with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, cycloalkyl with 3 to 7 carbon atoms, phenyl, phenoxy and alkoximinoalkyl with 1 to 4 carbon atoms in the alkoxy group and 1 to 4 carbon atoms in the alkyl group,

X represents hydrogen, fluorine, chlorine or bromine and

Y represents hydrogen, fluorine, chlorine or bromine,

characterized in that phenoxypropyl ketones of the formula

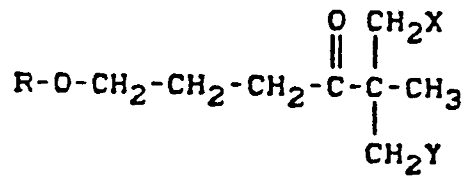

(IV)

in which

R, X and Y have the abovementioned meaning, are reacted with trimethylsulphonium iodide or trimethylsulphonium methosulphate in the presence of a base and in the presence of a diluent.

8. Phenoxy-substituted ketones of the formula

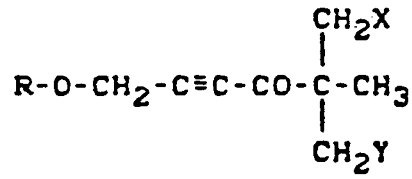

(VII)

in which

R represents phenyl which is optionally monosubstituted, disubstituted or trisubstituted by identical or different substituents, possible substituents being: halogen, alkyl with 1 to 4 carbon atoms, alkoxy with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, cycloalkyl with 3 to 7 carbon atoms, phenyl, phenoxy and alkoximinoalkyl with 1 to 4 carbon atoms in the alkoxy group and 1 to 4 carbon atoms in the alkyl group,

X represents hydrogen, fluorine, chlorine or bromine and

Y represents hydrogen, fluorine, chlorine or bromine.

9. Process for the preparation of phenoxypropyl ketones of the formula

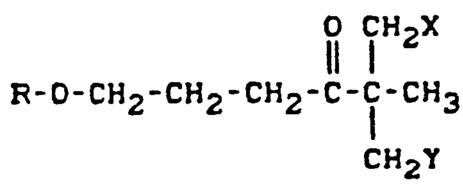

(IV)

in which

R represents phenyl which is optionally monosubstituted, disubstituted or trisubstituted by identical or different substituents, possible substituents being: halogen, alkyl with 1 to 4 carbon atoms, alkoxy with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, cycloalkyl with 3 to 7 carbon atoms, phenyl, phenoxy and alkoximinoalkyl with 1 to 4 carbon atoms in the alkoxy group and 1 to 4 carbon atoms in the alkyl group,

X represents hydrogen, fluorine, chlorine or bromine and

Y represents hydrogen, fluorine, chlorine or bromine,

characterized in that acid halides of the formula

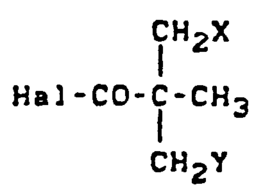

(V),

in which

X and Y have the abovementioned meaning and

Hal represents chlorine, bromine and iodine, are reacted with phenoxypropargyl compounds of the formula

$$R—O—CH_2—C{\equiv}C—H \qquad (VI)$$

in which

R has the abovementioned meaning, if appropriate in the presence of a diluent and if appropriate in the presence of an acid-binding agent and if appropriate in the presence of a catalyst, and the phenoxy-substituted ketones thereby formed, of the formula

$$R\text{-}O\text{-}CH_2\text{-}C{\equiv}C\text{-}CO\text{-}\underset{\substack{|\\CH_2Y}}{\overset{\substack{CH_2X\\|}}{C}}\text{-}CH_3 \qquad (VII)$$

in which

R, X and Y have the abovementioned meaning, are reacted with hydrogen in the presence of a hydrogenation catalyst and in the presence of a diluent.